# EUROPÄISCHE PATENTSCHRIFT

(11) **EP 0 483 536 B1**
(45) Veröffentlichungstag und Bekanntmachung des Hinweises auf die Patenterteilung: **11.01.1995**
(21) Anmeldenummer: 91116823.5
(22) Anmeldetag: 02.10.1991
(51) Int. Cl.: C07C 53/08, C07C 53/12, C07C 51/12, C07C 51/56

(54) **Kontinuierliches Verfahren zur gleichzeitigen Herstellung von Essigsäure und Essigsäuranhydrid**
Continuous process for the simultaneous preparation of acetic acid and acetic anhydride
Procédé continu pour la préparation simultanée de l'acide acétique et de l'anhydride acétique

(30) Priorität: 02.11.1990 DE 4034867
(43) Veröffentlichungstag der Anmeldung: 06.05.1992
(73) Patentinhaber: HOECHST AKTIENGESELLSCHAFT, 65926 Frankfurt am Main (DE)
(72) Erfinder: Erpenbach, Heinz, Dr., Köln (DE); Gradl, Reinhard, Dr., Erftstadt (DE); Jägers, Erhard, Dr., Bornheim (DE); Seidel, Andreas, Dr., Köln (DE)

(56) Entgegenhaltungen:
- EP-A- 0 170 964
- EP-A- 0 170 965
- DE-B- 1 966 695

## Beschreibung

Die Erfindung betrifft ein kontinuierliches Verfahren zur gleichzeitigen Herstellung von Essigsäure und Essigsäureanhydrid durch Umsetzung von Gemischen aus Methanol und Methylacetat sowie ggf. Dimethylether mit Kohlenmonoxid.

Essigsäure und Essigsäureanhydrid sind Zwischenprodukte bei den Vinylacetat- bzw. Celluloseacetatprodukten, die in steigendem Maße in der Technik hergestellt werden.

Aus der DE-3 823 645 C1 ist ein Verfahren zur gleichzeitigen Herstellung von Essigsäure und Essigsäureanhydrid durch eine Carbonylierung von Methanol und Methylacetat in Gegenwart eines Katalysatorsystems, das Carbonylkomplexe von Edelmetallen der Gruppe VIII des Periodensystems der Elemente enthält, bekannt. Insbesondere Rhodium ist als Katalysatorbestandteil von herausragender Wirksamkeit. In der Reaktionslösung wird die Rhodiumkonzentration auf 0,005 bis 0,05 mol/l, insbesondere 0,015 bis 0,027 mol/l, eingestellt. Die geringe Verfügbarkeit des Rhodiums bedingt jedoch ein unbefriedigend hohes Kostenniveau für dieses Verfahren.

Es hat deshalb nicht an Versuchen gefehlt, das teuere Rhodium durch einen preiswerteren Ersatzkatalysator auszutauschen.

Als Ersatzkatalysator wird Nickel für Carbonylierungen von Methylacetat oder Dimethylether zur Herstellung von Essigsäureanhydrid, beispielsweise in der DE-31 51 371 C2, vorgeschlagen. Nachteilig bei der Arbeit mit diesem Ersatzkatalysator sind die geringe Raum-Zeit-Ausbeute und die großen Nickelsalzkonzentrationen, die eingesetzt werden müssen, um technisch brauchbare Umsätze zu erzielen. Das führt insbesondere zu Schwierigkeiten, wenn die Carbonylierung als kontinuierliches Verfahren betrieben werden soll.

In der DE-27 49 955 C2 wird ebenfalls ein Nickel-Katalysator für Carbonylierungen von Methanol zur Herstellung von Essigsäure vorgeschlagen. Zur Erhaltung der Katalysatoraktivität bei kontinuierlichem Betrieb wird das Kohlenmonoxid zusammen mit Wasserstoff eingesetzt. Nachteilig hierbei ist, daß durch den Wasserstoffzusatz die Selektivität durch die Bildung von Ethylidendiacetat und die Raum-Zeit-Ausbeute bei gleichem CO-Gesamtdruck verschlechtert werden. Auch hier muß eine hohe Katalysatorkonzentration eingehalten werden, die beim kontinuierlichen Betrieb zu Schwierigkeiten führt.

Es war daher die Aufgabe gestellt, sowohl Essigsäure als auch Essigsäureanhydrid im gleichen Reaktionssystem in kontinuierlichen Verfahren herzustellen, wobei eine hohe Raum-Zeit-Ausbeute mit großer Selektivität bei mittleren Druckbereichen erreicht wird, geringe Korrosion am Reaktor eintritt und das Verfahren entsprechend den jeweiligen wirtschaftlichen Erfordernissen dem Mengenverhältnis von Essigsäure zu Essigsäureanhydrid in einfacher Weise angepaßt werden kann.

Die vorliegende Erfindung betrifft somit ein kontinuierliches Verfahren zur gleichzeitigen Herstellung von Essigsäure und Essigsäureanhydrid aus einem Ausgangsgemisch von Methanol und Methylacetat sowie ggf. Dimethylether durch Umsetzung mit Kohlenmonoxid unter wasserfreien Bedingungen bei Temperaturen von 150 bis 250 °C und erhöhtem Druck in einer Reaktionszone an einem gelösten Katalysatorsystem, enthaltend eine Nickelverbindung, Methyliodid als Promotor, mindestens einen Co-Promotor wie Alkaliiodid, quartäres Phosphonium- oder quartäres Ammoniumiodid, und einen Co-Katalysator, welches dadurch gekennzeichnet ist, daß man als Co-Katalysator eine Rhodium- oder Iridiumverbindung in einem molaren Mengenverhältnis Nickel : Co-Katalysator von 1 : (0,005 bis 0,1) verwendet, eine Nickelkonzentration in der Reaktionszone von 0,01 bis 0,1, vorzugsweise von 0,015 bis 0,06 mol/l einstellt und bei erhöhtem Druck von 30 bis 100, insbesondere von 50 bis 80 bar arbeitet.

Das Verfahren der Erfindung kann weiterhin bevorzugt dadurch gekennzeichnet sein, daß man in der Reaktionszone eine Verweilzeit von 1 bis 10 Minuten einstellt.

Bei dem erfindungsgemäßen Verfahren haben als Co-Promotoren insbesondere LiJ, NaJ, Methyltributyl-, Methyltriphenyl-, Tetrabutyl- oder Dimethyldibutylphosphoniumiodid sowie N,N-Dimethylimidazolium-, N-Methylpyridinium, N-Methyl-3-picolinium- oder N-Methylchinoliniumiodid in Mengen von 0,05 bis 4,5 mol/l Reaktionslösung gute Ergebnisse gebracht.

Das Verhältnis von Methanol zu Methylacetat oder Dimethylether kann in großen Grenzen gewählt werden. Für gute Raum-Zeit-Ausbeuten hat sich ein Molverhältnis von Methanol : Methylacetat und/oder Dimethylether von 10 : 1 bis 1 : 10 bewährt.

Kleinere Verunreinigungen des Kohlenmonoxids durch Wasserstoff, Wasserdampf sowie CO₂, CH₄ oder andere Inertgase sind ohne Bedeutung.

Temperaturen über 250 °C fördern die Bildung teeriger Bestandteile, die bei der Abtrennung des Katalysatorsystems von den Produkten stören.

Die Reaktionslösung enthält bevorzugt 0,1 bis 7,5 mol/l Methyliodid als Promotor.

Die Nickelverbindung kann als Nickelchlorid, -iodid, -acetat oder -acetylacetonat sowie als Nickeltetracarbonyl zugesetzt werden.

Geeignete Rhodium- und Iridiumverbindungen sind die entsprechenden Chloride, Iodide oder Komplexverbindungen wie beispielsweise
[CH₃P(C₄H₉)₃] Rh(CO)I₄ oder
[CH₃P(C₄H₉)₃] Rh(CO)₂I₂.

Mit dem erfindungsgemäßen Katalysatorsystem ist auch eine chargenweise Herstellung von Essigsäure und Essigsäureanhydrid möglich; jedoch entfaltet das erfindungsgemäße Katalysatorsystem seine guten Eigenschaften bei der kontinuierlichen Fahrweise. Bei der kontinuierlichen Fahrweise erhält das Katalysatorsystem über lange Arbeitsperioden seine volle Katalysatoraktivität hinsichtlich der Unterdrückung von Nebenreaktionen und großer Raum-Zeit-Ausbeuten. Es kommt zu keinen Salzablagerungen bei der kontinuierlichen Fahrweise. Durch den geringen Einsatz an Rhodium oder Iridium verbilligen sich die Katalysatorkosten wesentlich. Die wasserfreie Fahrweise bedingt nur einen unwesentlichen Eintrag von Korrosionsprodukten in die Reaktionslösung.

Obwohl das Katalysatorsystem auch zur Herstellung von höheren Alkansäuren und Alkansäureanhydriden einsetzbar ist, entfaltet es seine besten Ergebnisse bei der Herstellung von Essigsäure und Essigsäureanhydrid. Der Einsatz von Ethanol-Methylacetat- bzw. Methanol-Ethylacetat-Gemischen erlaubt auch die Herstellung von Gemischen aus Essigsäure/Propionsäure/Essigsäureanhydrid/Propionsäureanhydrid und dem gemischten Anhydrid von Essigsäure und Propionsäure.

### Beispiel 1

Die Carbonylierung wird bei einer Temperatur von 190 °C unter einem Druck von 70 bar in einem Reaktor mit 4,5 l Nutzvolumen durchgeführt. Die Reaktionslösung im Reaktor enthält im Mittel 0,060 mol/l Nickeliodid, 0,002 mol/l Rhodiumtrichlorid, 0,676 mol/l Methyltributylphosphoniumiodid und 2,3 mol/l Methyliodid.

Stündlich werden 0,79 kg (10,7 mol) Methylacetat, 1,67 kg (52,1 mol) Methanol und 11,7 kg Leichtsieder (Methylacetat, Methyliodid) über die Zugabeleitung 7 dem Reaktor 2 zugeführt. Durch Leitung 3 werden 1,76 kg/h (62,8 mol/h) Kohlenmonoxid in den Reaktor 2 gedrückt. Gleichzeitig werden über die Rückleitung 4 9,8 kg/h Destillationssumpf, in der das Katalysatorsystem gelöst ist, aus der Verdampfungsstufe 12 in den Reaktor 2 dosiert. Hieraus errechnet sich eine mittlere Verweilzeit im Reaktor 2 von 10 Minuten. Über die Produktleitung 5 werden 22,9 kg/h Reaktionslösung durch das Entnahmeventil 6 in flüssiger Phase dem Abscheider 8 zugeführt. Aus dem Abscheider 8, der unter einem Druck von 1 bar und 95 °C betrieben wird, fließt das flüssige Produkt durch Leitung 11 in die Verdampfungsstufe 12; die gasförmigen Anteile aus dem Abscheider 8 und der Verdampfungsstufe 12 strömen durch die Leitungen 9 und 13 in die Leichtsiederkolonne 10. Über die Abgasleitung 22 werden 0,07 kg/h Inertgase aus dem Kondensator 14 ausgeschleust. In der Leichtsiederkolonne 10 erfolgt bei einem Druck von 1 bar bei 126 °C Sumpf- und 70 °C Kopftemperatur die Abtrennung der Leichtsieder (Methyliodid, Methylacetat), welche aus dem Kondensator 14 über Leitung 1 in den Reaktor 2 rückgeführt werden. 4,22 kg/h Sumpfprodukt werden über die Ablaufleitung 15 in die Kolonne 16, die bei einem Druck von 0,15 bar bei 70 °C Kopf- und 99 °C Sumpftemperatur betrieben wird, gefördert. Als Kopfprodukt werden aus der Kolonne 16 3,07 kg/h reine Essigsäure über die Produktleitung 20 abgezogen. Hieraus errechnet sich eine Ausbeute von 98 %, bezogen auf eingesetztes Methanol.
Das Sumpfprodukt wird über die Ablaufleitung 17 in die Kolonne 18, die bei einem Druck von 0,15 bar bei 90 °C Kopf- und 104 °C Sumpftemperatur betrieben wird, überführt. Als Kopfprodukt werden der Kolonne 18 1,08 kg/h reines Essigsäureanhydrid über die Produktleitung 21 abgezogen. Hieraus errechnet sich eine Ausbeute von 99 %, bezogen auf eingesetztes Methylacetat.
0,07 kg/h Hochsieder werden als Sumpfprodukt der Kolonne 18 über die Sumpfleitung 19 entnommen.
Die Ausbeute an Essigsäure und Essigsäureanhydrid entspricht 98,1 %, bezogen auf den CO-Umsatz.
Die Raum-Zeit-Ausbeute beträgt 922 g Essigsäure und Essigsäureanhydrid/l . h.

### Beispiel 2

Das Beispiel 1 wurde abgewandelt, indem stündlich 0,79 kg (10,7 mol) Methylacetat, 2,43 kg (75,9 mol) Methanol über die Zugabeleitung 7 und 2,42 kg/h CO über Leitung 3 dosiert wurden.
Hieraus errechnet sich eine Verweilzeit von 9 Minuten im Reaktor 2.
Die Ausbeute an Essigsäure und Essigsäureanhydrid entspricht 98 %, bezogen auf den CO-Umsatz.
Es wurden 4,49 kg/h reine Essigsäure und 1,07 kg/h reines Essigsäureanhydrid erhalten.
Die Raum-Zeit-Ausbeute beträgt 1236 g Essigsäure und Essigsäureanhydrid/l . h.

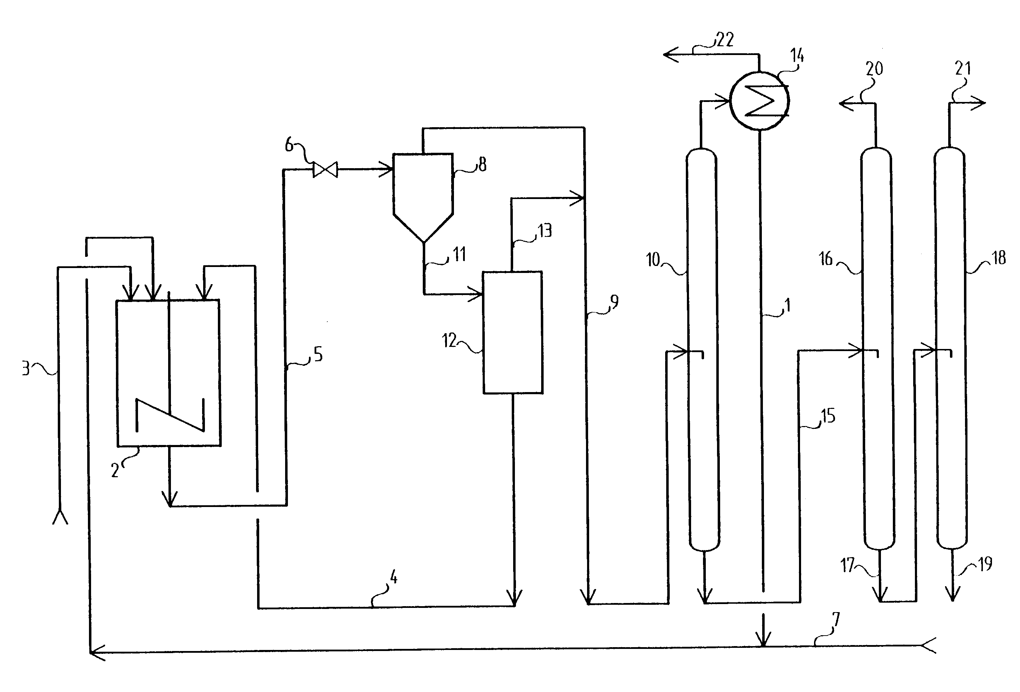

## Patentansprüche

1. Kontinuierliches Verfahren zur gleichzeitigen Herstellung von Essigsäure und Essigsäureanhydrid aus einem Ausgangsgemisch von Methanol und Methylacetat sowie gegebenenfalls Dimethylether durch Umsetzung mit Kohlenmonoxid unter wasserfreien Bedingungen bei Temperaturen von 150 bis 250 °C und erhöhtem Druck in einer Reaktionszone an einem gelösten Katalysatorsystem, enthaltend eine Nickelverbindung, Methyliodid als Promotor, mindestens einen Co-Promotor wie Alkaliiodid, quartäres Phosphonium- oder quartäres Ammoniumiodid, und einen Co-Katalysator, dadurch gekennzeichnet, daß man als Co-Katalysator eine Rhodium- oder Iridiumverbindung in einem molaren Mengenverhältnis Nickel : Co-Katalysator von 1 : (0,005 bis 0,1) verwendet, eine Nickelkonzentration in der Reaktionszone von 0,01 bis 0,1, vorzugsweise von 0,015 bis 0,06 mol/l einstellt und bei erhöhtem Druck von 30 bis 100, insbesondere von 50 bis 80 bar arbeitet.

2. Verfahren nach Anspruch 1, dadurch gekennzeichnet, daß man in der Reaktionszone eine Verweilzeit von 1 bis 10 Minuten einstellt.

## Claims

1. A continuous process for the simultaneous preparation of acetic acid and acetic anhydride from a starting mixture of methanol and methyl acetate and also, optionally, dimethyl ether, by reaction with carbon monoxide under anhydrous conditions at temperatures of 150 to 250°C and under elevated pressure in a reaction zone on a dissolved catalyst system containing a nickel compound, methyl iodide as promoter, at least one co-promoter, such as an alkali metal iodide, quaternary phosphonium iodide or quaternary ammonium iodide, and a co-catalyst, which process comprises using, as co-catalyst, a rhodium or iridium compound in a molar ratio of nickel: co-catalyst of 1: (0.005 to 0.1), adjusting the nickel concentration in the reaction zone to 0.01 to 0.1, preferably to 0.015 to 0.06 mol/l, and operating under an elevated pressure of 30 to 100 bar, in particular of 50 to 80 bar.

2. The process as claimed in claim 1, wherein the residence time in the reaction zone is adjusted to 1 to 10 minutes.

## Revendications

1. Procédé continu de préparation simultanée d'acide acétique et d'anhydride acétique à partir d'un mélange de départ constitué de méthanol et d'acétate de méthyle ainsi que le cas échéant d'éther diméthylique, par réaction avec du monoxyde de carbone dans des conditions anhydres à des températures comprises entre 150 à 250°C et sous pression élevée dans une zone réactionnelle sur un système catalytique dissous, contenant un composé du nickel, de l'iodure de méthyle en tant que promoteur, au moins un co-promoteur comme un iodure d'alcalin, un iodure de phosphonium quaternaire ou un iodure d'ammonium quaternaire et un co-catalyseur, caractérisé en ce qu'on utilise comme co-catalyseur un composé du rhodium ou de l'iridium en un rapport quantitatif molaire nickel : co-catalysateur de 1 : (0,005 à 0,1), qu'on fixe une concentration de nickel dans la zone réactionnelle comprise entre 0,01 et 0,1, de préférence 0,015 et 0,06 mol/l et qu'on travaille sous une pression élevée comprise entre 30 et 100, de préférence 50 et 80 bar.

2. Procédé selon la revendication 1, caractérisé en ce qu'on fixe un temps de séjour dans la zone réactionnelle compris entre 1 et 10 minutes.
